(19) Europäisches Patentamt
European Patent Office
Office européen des brevets

(11) Numéro de publication : **0 079 810**
**B1**

(12) **FASCICULE DE BREVET EUROPÉEN**

(45) Date de publication du fascicule du brevet : **22.01.86**

(21) Numéro de dépôt : **82401905.3**

(22) Date de dépôt : **18.10.82**

(51) Int. Cl.⁴ : **C 07 D239/84**, C 07 D401/04, A 61 K 31/505 // (C07D401/04, 239:84, 211:22, 211:42, 211:44)

(54) **Nouveaux dérivés de la phényl-4 quinazoline actifs sur le système nerveux central.**

(30) Priorité : **21.10.81 FR 8119767**

(43) Date de publication de la demande : **25.05.83 Bulletin 83/21**

(45) Mention de la délivrance du brevet : **22.01.86 Bulletin 86/04**

(84) Etats contractants désignés : **AT BE CH DE FR GB IT LI LU NL SE**

(56) Documents cités : **FR-A- 2 125 378** **US-A- 3 509 141**

(73) Titulaire : **SANOFI, société anonyme**
**40, Avenue George V**
**F-75008 Paris (FR)**

(72) Inventeur : **Biziere, Kathleen**
**6 Lotissement Rayons d'Oc**
**F-34170 Clapiers (FR)**

(74) Mandataire : **Gillard, Marie-Louise**
**Cabinet Beau de Loménie 55, Rue d'Amsterdam**
**F-75008 Paris (FR)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

EP 0 079 810 B1

**Description**

La présente invention concerne en tant que produits industriels nouveaux des dérivés de la chloro-6 phényl-4 quinazoline substitués en position 2 par un groupement hydroxyamino ainsi que leur méthode de préparation et leur application comme médicaments.

Des dérivés de la chloro-6 phényl-4 quinazoline substitués en position 2 par un groupe amino secondaire ou tertiaire non hydroxylé, et présentant une activité antiinflammatoire sont décrits dans le brevet US 3 509 141.

On a maintenant trouvé des dérivés de la chloro-6 phényl-4 quinazoline substitués en position 2 par un groupement hydroxylamino, actifs sur le système nerveux central.

Les composés selon l'invention répondent à la formule générale :

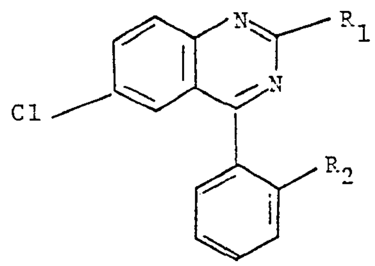

(I)

dans laquelle :

$R_1$ peut désigner soit :

a) un groupe

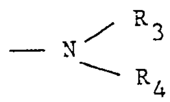

dans lequel $R_3$ représente l'hydrogène ou un groupe $-(CH_2)_nOH$ et $R_4$ représente un groupe $(CH_2)_nOH$ où n désigne un nombre entier variant de 2 à 4 ;

b) un groupe

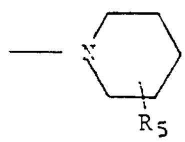

où $R_5$ désigne un groupe $-(CH_2)_m-OH$ (m étant un entier pouvant varier 0 à 2) situé en position méta ou para ;

c) un groupe

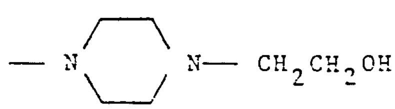

$R_2$ représente un atome d'halogène de préférence le chlore ou le fluor ou encore $R_2$ peut être l'hydrogène dans le cas où $R_1$ est

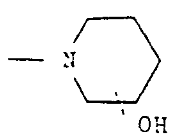

Les composés (I) fournissent avec les acides minéraux ou organiques des sels solubles. Ces sels, avec des acides pharmaceutiquement acceptables, font partie intégrante de l'invention.

Les composés (I) peuvent être préparés à partir de la chloro-6 phényl-4 quinazolone-2 convenablement substituée (1) suivant le schéma réactionnel :

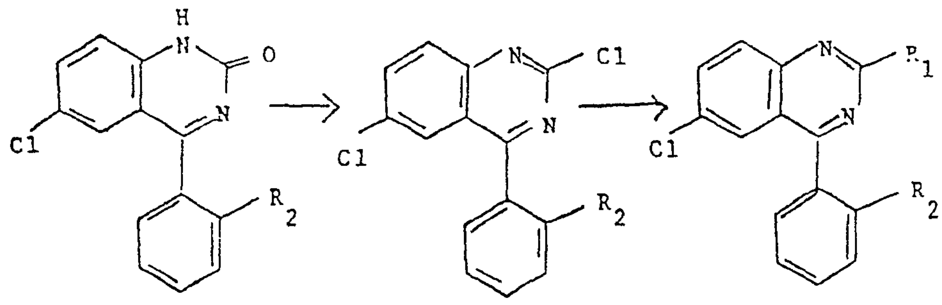

(1) (2) (I)

Par action sur la quinazolone 1 d'un dérivé chloré du phosphore, on obtient le dérivé 2 chloré en position 2. On utilise le plus souvent l'oxychlorure de phosphore. On peut opérer au sein d'un solvant inerte tel qu'un hydrocarbure aromatique (benzène ou toluène) mais le plus souvent on préfère utiliser un excès d'oxychlorure comme solvant. La réaction a lieu à une température comprise entre 60 et 120 °C et le plus souvent à la température d'ébullition du solvant utilisé.

A partir du dérivé chloré 2, par action d'une amine $R_1H$ en excès au sein d'un solvant inerte comme l'éthanol on obtient le composé (I) correspondant. On opère en général par chauffage à la température d'ébullition du solvant.

Les sels des composés (I) sont obtenus de façon habituelle en salifiant la base à chaud par une quantité stœchiométrique d'acide au sein d'un solvant convenablement choisi de façon à ce que le sel formé cristallise par refroidissement.

Les quinazolones de départ 1 sont des composés connus qui peuvent en particulier être préparés par action du cyanate de potassium sur une amino-2 chloro-5 benzophénone convenablement substitué.

Les exemples suivants sont donnés à titre d'illustration pour la préparation des composés (I) suivant la méthode indiquée précédemment.

## Exemple 1

Chloro-6 (chloro-2 phényl)-4 (hydroxy-4 pipéridinyl-1)-2 quinazoline, chlorhydrate (CM 40331).

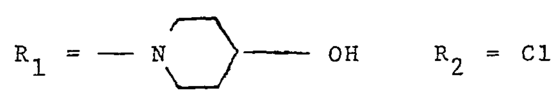

(I)

a) Dichloro-2,6 (chloro-2 phényl)-4 quinazoline.

On chauffe à reflux pendant 6 heures sous agitation, le mélange de 11,8 g de chloro-6 (chloro-2 phényl)-4 quinazolone-2 et 59 ml d'oxychlorure de phosphore. On évapore sous vide à siccité l'excès d'oxychlorure de phosphore et verse le résidu sur un mélange glace-eau. On essore le solide qui se sépare, lave avec de l'eau puis recristallise dans l'éthanol.

On obtient 11,7 g du produit attendu, F : 175-6 °C.

b) CM 40 331 :

On chauffe au reflux pendant 1 heure, le mélange de 13,1 g du produit obtenu ci-dessus et 12,8 g d'hydroxy-4 pipéridine dans 68 ml d'éthanol. On évapore le solvant et reprend le résidu dans l'eau puis on extrait avec de l'acétate d'éthyle. On sèche la solution organique sur sulfate de sodium et évapore le solvant à siccité. On dissout le résidu dans l'éther isopropylique bouillant et laisse cristalliser par refroidissement.

On obtient ainsi 10,5 de base, F : 129-131 °C.

Chlorhydrate :

On dissout la base obtenue ci-dessus (10,5 g) dans 70 ml d'éthanol absolu et ajoute de l'acide chlorhydrique gazeux jusqu'à pH acide. On essore les cristaux formés et recristallise dans l'éthanol absolu. Poids 10,6 g ; F : 212-4 °C

## Exemples 2 à 11

a) Dichloro-2,6 ($R_2$-2 phényl)-4 quinazoline.

En opérant comme dans l'exemple 1 a) mais en faisant varier la quinazolone de départ on obtient de la même façon :

la dichloro-2,6 (fluoro-2 phényl)-4 quinazoline ;
F : 208-210 °C (acétonitrile),
la dichloro-2,6 phényl-4 quinazoline ;
F : 159-160 °C (acétate d'éthyle).

b) A partir des divers dérivés chlorés mentionnés précédemment et en faisant varier l'amine $R_1H$ utilisée, on obtient, comme indiqué dans l'exemple 1 b), les différents composés (I) réunis dans le tableau I.

Les produits selon l'invention ont été soumis à des essais pharmacologiques en vue de déterminer leur activité sur le système nerveux central.

1) Action anticonvulsivante.

On a en particulier étudié l'action anticonvulsivante des produits selon l'invention par le test de l'antagoniste des convulsions induites par le pentétrazol.

Ce test a été réalisé sur des souris femelles naïves, OF1 (Iffa Credo, France) pesant 18 à 23 g.

La veille de l'expérience, les animaux sont marqués, pesés, placés par 10 dans des cages de macrolon (30 × 19 × 12 cm) et maintenus dans l'animalerie climatisée jusqu'au jour de l'expérience. La nourriture et la boisson sont administrées ad libitum. Le jour de l'expérience, les animaux sont placés dans le laboratoire où doit avoir lieu l'expérience.

Les produits à étudier sont mis en suspension dans l'eau gommée à 5 % et administrés oralement à la dose de 200 mg/kg (exprimée en sel) à des lots de 10 souris sous un volume de 20 ml/kg. Les témoins reçoivent l'eau gommée seule.

Une heure après l'administration des produits à étudier, le pentétrazol dissous dans l'eau distillée, est administré par voie intrapéritonéale à la dose de 125 mg/kg sous un volume de 20 ml/kg. Immédiatement après l'injection du convulsivant, les souris sont placées dans des récipients individuels (10 × 10 × 15 cm). Une heure après l'administration de pentétrazol la mortalité est relevée.

Les résultats sont exprimés en pourcentage d'animaux ayant survécu, c'est-à-dire en pourcentage d'animaux ayant été protégés de l'action du pentétrazol.

2) Potentialisation de la narcose induite par le pentobarbital.

Ce test a été réalisé sur des souris femelles naïves, OF1 (Iffa-Credo, France) pesant 18 à 23 g.

La veille de l'expérience, les animaux sont marqués, pesés, placés par 10 dans des cages macrolon (30 × 19 × 12 cm) et maintenus dans l'animalerie climatisée jusqu'au jour de l'expérience. La nourriture et la boisson sont administrées ad libitum. Le jour de l'expérience, les animaux sont placés dans le laboratoire où doit avoir lieu l'expérience.

Les produits à étuidier sont mis en suspension dans l'eau gommée à 5 % et administrés oralement à la dose de 120 mg/kg (exprimée en sel) à des lots de 10 souris sous un volume de 20 ml/kg. Les témoins reçoivent l'eau gommée seule.

Une heure après l'administration des produits à étudier, le pentobarbital (pentobarbital sodique à 6 %) est administré à la dose de 20 mg/kg sous un volume de 20 ml/kg. On note le nombre d'animaux en perte de « righting reflex ». Les résultats sont exprimés en % d'animaux qui dorment.

Les résultats obtenus sur ces 2 tests avec les produits de l'invention sont réunis dans le tableau II.

Par ailleurs les produits de l'invention sont peu toxiques. La totalité des produits essayés sont totalement atoxiques à la dose de 500 mg/kg.

Aussi ces produits peuvent-ils être utilisés en thérapeutique humaine comme anxiolytiques, hypnotiques et antiépileptiques.

Les produits pourront être conditionnés sous les formes galéniques correspondant à la voie orale (poudres, comprimés, gélules...) et à la voie parentérale (ampoules injectables).

La posologie variable suivant les affections à traiter et la voie d'administration, sera progressive et se situera entre 100 et 600 mg par jour chez l'adulte.

A titre d'exemple, on peut indiquer la préparation galénique suivante :

| | |
|---|---|
| Gélule | |
| CM 40 331 | 0,050 g |
| Amidon STA RX 1 500 | 0,048 g |
| Stéarate de magnésium | 0,002 g |
| pour 1 gélule n° 3. | |

(Voir Tableau 1 page 5)

Tableau I

| Exemple N° | N° de Code du Produit | $R_1$ | $R_2$ | Base ou Sel Point de fusion °C (solvant de recristallisation) |
|---|---|---|---|---|
| 2 | 40 332 | 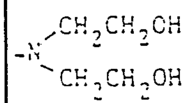 | Cl | Chlorhydrate 176 - 8 (isopropanol) |
| 3 | 40 411 | " " | F | Base 144-6 (méthanol) Chlorhydrate 194-6 (isopropanol) |
| 4 | 40 416 | 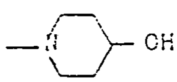 | F | Base 188-190 (méthanol) |
| 5 | 40 451 | $-NH-CH_2CH_2OH$ | Cl | Base 144-6 (acétate d'éthyle) |
| 6 | 40 621 | $-NH-CH_2CH_2CH_2OH$ | Cl | Base 124-6 (éther) |
| 7 | 40 669 | 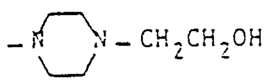 | CL | Base 118-120 (éther isopropylique) |
| 8 | 40 744 | $-NH-(CH_2)_3CH_2OH$ | Cl | Base 126-8 (acétate d'éthyle) |
| 9 | 40 785 | 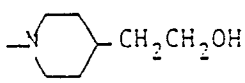 | Cl | Chlorhydrate 192-5 (isopropanol) |
| 10 | 40 788 | 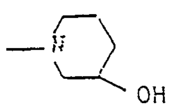 | Cl | Chlorhydrate 210-2 (isopropanol) |
| 11 | 40 854 | 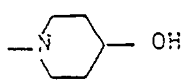 | H | Chlorhydrate 260-2 (éthanol à 96) |

Tableau II

| N° de Code du Produit | Antagonisme du Cardiazol (% d'animaux protégés) | Narcose % d'animaux qui dorment |
|---|---|---|
| 40 331 | 100 | 100 |
| 40 788 | 50 | 100 |
| 40 332 | 50 | 80 |

(suite)

| N° de Code du Produit | Antagonisme du Cardiazol (% d'animaux protégés) | Narcose % d'animaux qui dorment |
|---|---|---|
| 40 451 | 50 | 50 |
| 40 785 | 30 | 80 |
| 40 744 | 40 | 60 |
| 40 854 | 30 | 20 |
| 40 416 | 20 | 20 |
| 40 669 | 30 | 40 |
| 40 411 | 50 | 50 |
| 40 621 | 20 | 70 |

**Revendications** (pour les Etats contractants : BE, CH, DE, FR, GB, IT, LI, LU, NL, SE)

1. Dérivés de la phényl-4 quinazoline, caractérisés en ce qu'ils répondent à la formule générale :

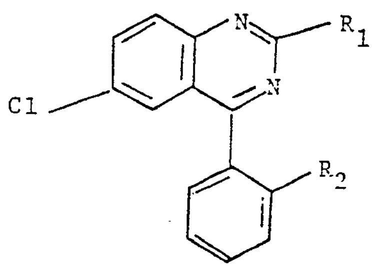

(I)

dans laquelle

$R_1$ est choisi parmi

a) un groupe

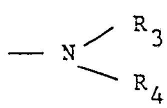

dans lequel $R_3$ représente l'hydrogène ou un groupe $-(CH_2)_nOH$ et $R_4$ représente un groupe $(CH_2)_nOH$ où n désigne un nombre entier égal à 2, 3 ou 4 ;

b) un groupe

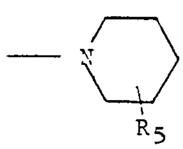

où $R_5$ désigne un groupe $-(CH_2)_m-OH$ (m étant un entier pouvant varier de 0 à 2) situé en position méta ou para ;

c) un groupe

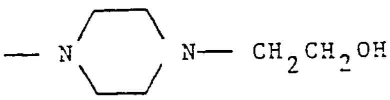

$R_2$ représente un atome d'halogène de préférence le chlore ou le fluor, ou encore $R_2$ peut être l'hydrogène dans le cas où $R_1$ est

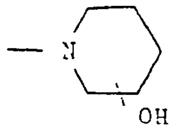

ainsi que les sels de ces nouveaux dérivés avec des acides minéraux ou organiques pharmaceutiquement acceptables.

2. Procédé de préparation des produits selon la revendication 1, caractérisé en ce qu'on utilise comme produit de départ une chloro-6 phényl-4 quinazolone-2 convenablement substituée, que l'on transforme ce produit de départ en une dichloro-2,6 phényl-4 quinazoline correspondante par action d'un dérivé chloré du phosphore à une température comprise entre environ 60 et environ 120 °C et que l'on fait réagir le produit obtenu avec une amine de formule $R_1H$, la réaction étant effectuée, avec un excès d'amine, dans un solvant inerte.

3. Procédé selon la revendication 2, caractérisé en ce que le dérivé chloré du phosphore est de l'oxychlorure de phosphore.

4. Procédé selon l'une des revendications 2 et 3, caractérisé en ce que la réaction avec l'amine est effectuée dans l'éthanol et à la température d'ébullition de ce solvant.

5. Médicaments utilisables notamment comme anxiolytiques, hypnotiques et antiépileptiques, caractérisés en ce qu'ils contiennent au moins un produit selon la revendication 1.

6. Médicaments selon la revendication 5, caractérisés en ce qu'ils sont conditonnés pour être administrés par voie orale ou par voie parentérale.

**Revendications** (pour l'Etat contractant AT)

1. Procédé de préparation de dérivés de la phényl-4 quinazoline répondant à la formule générale :

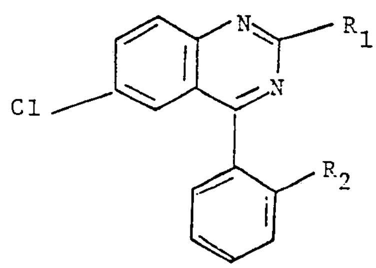

(I)

dans laquelle

$R_1$ est choisi parmi

a) un groupe

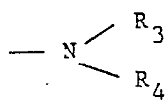

dans lequel $R_3$ représente l'hydrogène ou un groupe $-(CH_2)_nOH$ et $R_4$ représente un groupe $(CH_2)_nOH$ où n désigne un nombre entier égal à 2, 3 ou 4 ;

b) un groupe

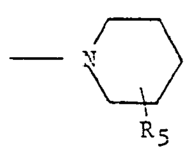

où $R_5$ désigne un groupe $-(CH_2)_m-OH$ (m étant un entier pouvant varier de 0 à 2) situé en position méta ou para ;

c) un groupe

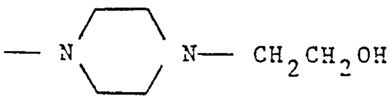

$R_2$ représente un atome d'halogène de préférence le chlore ou le fluor, ou encore $R_2$ peut être l'hydrogène dans le cas où $R_1$ est

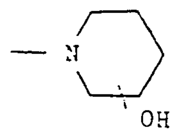

et des sels desdits dérivés avec des acides minéraux ou organiques pharmaceutiquement acceptables, caractérisé en ce que l'on utilise comme produit de départ une chloro-6 phényl-4 quinazolone-2 convenablement substituée, que l'on transforme ledit produit de départ en une dichloro-2,6 phényl-4 quinazoline correspondante par action d'un dérivé chloré du phosphore à une température comprise entre environ 60 °C et environ 120 °C puis que l'on fait réagir le produit obtenu avec une amine de formule $R_1H$, dans laquelle $R_1$ est tel que défini ci-dessus, la réaction étant effectuée avec un excès d'amine, dans un solvant inerte, et qu'éventuellement le produit obtenu sous forme de base est salifié.

2. Procédé selon la revendication 1, caractérisé en ce que le dérivé chloré du phosphore est de l'oxychlorure de phosphore.

3. Procédé selon l'une des revendications 1 ou 2, caractérisé en ce que la réaction avec l'amine est effectuée dans l'éthanol et à la température d'ébullition de ce solvant.

4. Utilisation des dérivés de la phényl-4 quinazoline de formule (I) selon la revendication 1, pour la préparation d'un médicament actif sur le système nerveux central.

**Claims** (for the Contracting States : BE, CH, DE, FR, GB, IT, LI, LU, NL, SE)

1. Derivatives of 4-phenyl quinazoline, characterized in that they respond to the general formula :

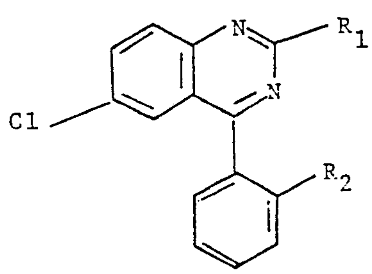

(I)

in which

$R_1$ is selected from

a) a group

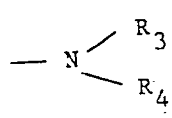

in which $R_3$ represents hydrogen or a group $-(CH_2)_nOH$ and $R_4$ represents a group $-(CH_2)_nOH$ wherein n designates an integer equal to 2, 3 or 4 ;

b) a group

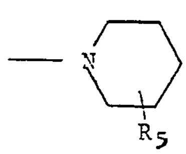

wherein $R^5$ designates a group $-(CH_2)_m-OH$ (m being an integer which may vary from 0 to 2) in the meta or para position ;

c) a group

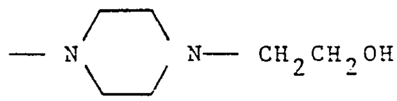

$R_2$ represents an atom of halogen, preferably chlorine of fluorine, or $R_2$ may be hydrogen in the case of $R_1$ being

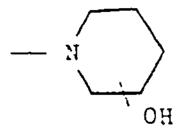

as well as the salts of these novel derivatives with pharmaceutically acceptable mineral or organic acids.

2. Process for preparing the products according to claim 1, characterized in that it comprises the steps of : using as starting product a suitably substituted 6-chloro 4-phenyl 2-quinazolone, converting this starting product into a corresponding 2,6-dichloro 4-phenyl quinazoline by action of a chlorinated derivative of phosphorus at a temperature of between about 60 and about 120 °C, and reacting the product obtained with an amine of formula $R_1H$, the reaction being carried out, with an excess of amine, in an inert solvent.

3. The process according to claim 2, characterized in that the chlorinated derivative of phosphorus is phosphorus oxychloride.

4. The process according to any one of claims 2 and 3, characterized in that the reaction with amine is carried out in ethanol and at the boiling temperature of this solvent.

5. Drugs which may be used in particular as minor tranquilizers, hypnotics and antiepileptics, characterized in that they contain at least one product according to claim 1.

6. The drugs according to claim 5, characterized in that they are packed to be administered by the oral or parenteral route.

**Claims** (for the Contracting State AT)

1. Process for preparing derivatives of 4-phenyl quinazoline, responding to the general formula :

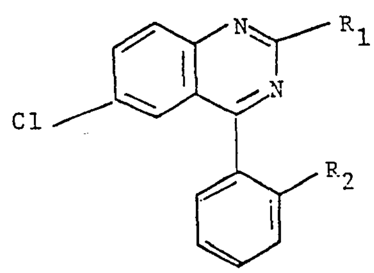

(I)

in which

$R_1$ is selected from

a) a group

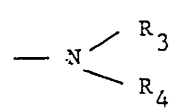

in which $R_3$ represents hydrogen or a group $-(CH_2)_nOH$ and $R_4$ represents a group $-(CH_2)_n-OH$ wherein n designates an integer equal to 2, 3 or 4 ;

b) a group

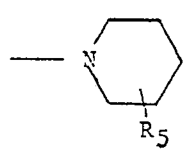

wherein $R_5$ designates a group $-(CH_2)_m-OH$ (m being an integer which may vary from 0 to 2) in the meta or para position ;

c) a group

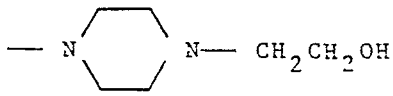

$R_2$ represents an atom of halogen, preferably chlorine of fluorine, or $R_2$ may be hydrogen in the case of $R_1$ being

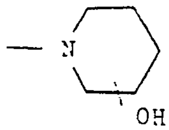

as well as the salts of these derivatives with pharmaceutically acceptable mineral or organic acids, characterized in that a suitably substituted 6-chloro 4-phenyl 2-quinazolone, is used as starting product, this starting product is converted into a corresponding 2,6-dichloro 4-phenyl quinazoline by action of a chlorinated derivative of phosphorus at a temperature of between about 60 and about 120 °C, and the product obtained is caused to react with an amine of formula $R_1H$, in which $R_1$ is as defined above, the reaction being carried out, with an excess of amine, in an inert solvent, and in that the product obtained in the form of a base is optionally converted into a salt.

2. The process according to claim 1, characterized in that the chlorinated derivative of phosphorus is phosphorus oxychloride.

3. The process according to any one of Claims 1 or 2, characterized in that the reaction with amine is carried out in ethanol and at the boiling temperature of this solvent.

4. Use of derivatives of 4-phenyl 2-quinazoline of formula (I) according to claim 1, for the preparation of a drug active on the central nervous system.

**Patentansprüche** (für die Vertragsstaaten : BE, CH, DE, FR, GB, IT, LI, LU, NL, SE)

1. 4-Phenyl-chinazolin-Derivate, dadurch gekennzeichnet, daß sie der allgemeinen Formel

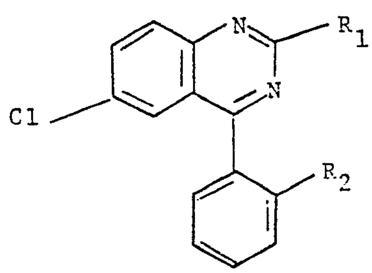

(I)

entsprechen, worin

$R_1$ ausgewählt ist aus

a) einer Gruppe

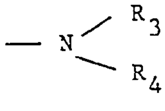

worin $R_3$ für Wasserstoff oder eine Gruppe $-(CH_2)_nOH$ steht und $R_4$ eine Gruppe $(CH_2)_nOH$ darstellt, wobei n eine ganze Zahl gleich 2, 3 oder 4 bedeutet ;

b) einer Gruppe,

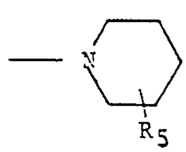

worin $R_5$ eine Gruppe $-(CH_2)_m-OH$ darstellt (wobei m eine ganze Zahl ist, die von 0 bis 2 variieren kann), die sich in der meta- oder para-Position befindet ;

c) einer Gruppe

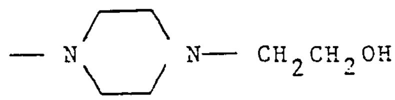

und $R_2$ für ein Halogenatom, vorzugsweise Chlor oder Fluor, steht oder Wasserstoff sein kann, wenn $R_1$

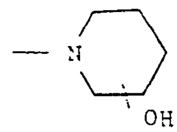

ist,
sowie die Salze dieser neuen Derivate mit pharmazeutisch akzeptablen Mineral- oder organischen Säuren.

2. Verfahren zur Herstellung der Produkte nach Anspruch 1, dadurch gekennzeichnet, daß man als Ausgangsprodukt ein entsprechend substituiertes 6-Chlor-4-phenyl-chinazol-2-on verwendet, daß man dieses Ausgangsprodukt durch Einwirken eines chlorierten Phosphorderivats bei einer Temperatur zwischen etwa 60 und etwa 120 °C in ein entsprechendes 2,6-Dichlor-4-phenyl-chinazolin überführt, und daß man das erhaltene Produkt mit einem Amin der Formel $R_1H$ reagieren läßt, wobei die Reaktion mit einem Überschuß an Amin in einem inerten Lösungsmittel durchgeführt wird.

3. Verfahren nach Anspruch 2, dadurch gekennzeichnet, daß das chlorierte Phosphorderivat Phosphoroxychlorid ist.

4. Verfahren nach einem der Ansprüche 2 und 3, dadurch gekennzeichnet, daß die Umsetzung mit dem Amin in Äthanol und bei der Kochtemperatur dieses Lösungsmittels durchgeführt wird.

5. Insbesondere Als Anxiolytika, Hypnotika und Antiepileptika nützliche Medikamente, dadurch gekennzeichnet, daß sie mindestens ein Produkt nach Anspruch 1 enthalten.

6. Medikamente nach Anspruch 5, dadurch gekennzeichnet, daß sie zur Verabreichung auf oralem Weg oder auf parenteralem Weg oder auf parenteralem Weg konditioniert sind.

**Patentansprüche** (für den Vertragsstaat AT)

1. Verfahren zur Herstellung von 4-Phenyl-chinazolin-Derivaten der allgemeinen Formel

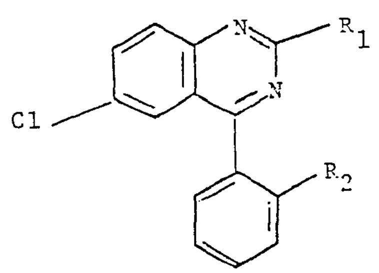

(I)

worin

$R_1$ ausgewählt ist aus

a) einer Gruppe

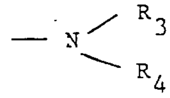

worin $R_3$ für Wasserstoff oder eine Gruppe $-(CH_2)_nOH$ steht und $R_4$ eine Gruppe $(CH_2)_nOH$ darstellt, wobei n eine ganze Zahl gleich 2, 3 oder 4 bedeutet ;

b) einer Gruppe,

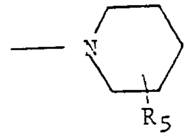

worin $R_5$ eine Gruppe $-(CH_2)_m-OH$ darstellt (wobei m eine ganze Zahl ist, die von 0 bis 2 variieren kann), die sich in der meta- oder para-Position befindet ;

c) einer Gruppe

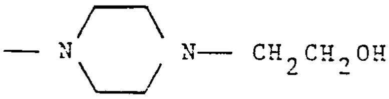

und $R_2$ für ein Halogenatom, vorzugsweise Chlor oder Fluor, steht oder Wasserstoff sein kann, wenn $R_1$

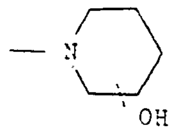

ist,

sowie der Salze dieser neuen Derivate mit pharmazeutisch akzeptablen Mineral- oder organischen Säuren, dadurch gekennzeichnet, daß man als Ausgangsprodukt ein entsprechend substituiertes 6-Chlor-4-phenyl-chinazol-2-on verwendet, daß man dieses Ausgangsprodukt durch Einwirken eines chlorierten Phosphorderivats bei einer Temperatur zwischen etwa 60 und etwa 120 °C in ein entsprechendes 2,6-Dichlor-4-phenyl-chinazolin überführt, daß man darauf das erhaltene Produkt mit einem Amin der Formel $R_1H$, worin $R_1$ obige Bedeutung hat, reagieren läßt, wobei die Reaktion mit einem Überschuß an Amin in einem inerten Lösungsmittel durchgeführt wird, und daß gegebenenfalls das in Basenform erhaltene Produkt in ein Salz übergeführt wird.

2. Verfahren nach Anspruch 1, dadurch gekennzeichnet, daß das chlorierte Phosphorderivat Phosphoroxychlorid ist.

3. Verfahren nach einem der Ansprüche 1 oder 2, dadurch gekennzeichnet, daß die Umsetzung mit dem Amin in Äthanol und bei der Kochtemperatur dieses Lösungsmittels durchgeführt wird.

4. Verwendung der 4-Phenyl-chinazolin-Derivate der Formel (I) nach Anspruch 1 zur Herstellung eines auf das Zentralnervensystem wirksamen Medikaments.